# EUROPEAN PATENT SPECIFICATION

(11) **EP 4 122 468 B1**
(45) Date of publication and mention of the grant of the patent: **07.10.2026**
(21) Application number: 21792591.6
(22) Date of filing: 23.04.2021
(51) Int. Cl.: A01N 31/06, A01N 35/06, A01N 37/02, A01N 43/16, A01N 43/90, A01N 45/00, A01P 7/02, A61K 31/56, A61K 31/352, A61K 31/7048, A61P 33/14

(54) **APPLICATION OF COMPOUNDS IN CONTROLLING OR KILLING MITES**
VERWENDUNG VON VERBINDUNGEN ZUR BEKÄMPFUNG ODER ABTÖTUNG VON MILBEN
APPLICATION DE COMPOSÉS DANS LA LUTTE CONTRE LES ACARIENS OU LA DESTRUCTION DE CES DERNIERS

(30) Priority: 24.04.2020 CN 202010334025
(43) Date of publication of application: 25.01.2023
(73) Proprietor: Wenzhou PrecVision Biotechnologies Ltd., Wenzhou City, Zhejiang 325000, (CN)
(72) Inventor: WEI, Lai, Wenzhou City 325000 (CN); ZHANG, Yan, Zhuhai, Guangdong 519000 (CN)
(74) Representative: J A Kemp LLP
(86) International application number: PCT/CN2021/089105
(87) International publication number: WO 2021/213483

(56) References cited:
- EP-A1- 4 129 314
- EP-A1- 4 129 316
- CN-A- 101 692 822
- CN-A- 101 731 215
- CN-A- 101 731 215
- CN-A- 103 432 312
- CN-A- 104 042 804
- CN-A- 108 651 465
- CN-A- 110 804 499
- CN-A- 110 946 815
- FLÃVIO GAIETA HOLZCHUH ET AL: "Clinical Treatment of Ocularby Systemic Ivermectin", AMERICAN JOURNAL OF OPHTHALMOLOGY, ELSEVIER, AMSTERDAM, NL, vol. 151, no. 6, 22 November 2010 (2010-11-22), pages 1030 - 1034.e1, XP028219023, ISSN: 0002-9394, [retrieved on 20101126], DOI: 10.1016/J.AJO.2010.11.024
- FROMSTEIN STEPHANIE R ET AL: "Demodex blepharitis: clinical perspectives", vol. Volume 10, 4 July 2018 (2018-07-04), pages 57 - 63, XP093071804, Retrieved from the Internet <URL:https://www.ncbi.nlm.nih.gov/pmc/articles/PMC6118860/pdf/opto-10-057.pdf> DOI: 10.2147/OPTO.S142708
- DATABASE CAS Registry [online] Chemical Abstracts Service; 16 November 1984 (1984-11-16), .: "Specific compounds of EP21792591", XP093071793, retrieved from STN Database accession no. various
- DUAN D. D. ET AL: "Isolation and Identification of Acaricidal Compounds in Inula japonica (Asteraceae)", vol. 104, no. 2, 1 April 2011 (2011-04-01), US, pages 375 - 378, XP055860049, ISSN: 0022-0493, Retrieved from the Internet <URL:https://watermark.silverchair.com/jee104-0375.pdf?token=AQECAHi208BE49Ooan9kkhW_Ercy7Dm3ZL_9Cf3qfKAc485ysgAAAtkwggLVBgkqhkiG9w0BBwagggLGMIICwgIBADCCArsGCSqGSIb3DQEHATAeBglghkgBZQMEAS4wEQQM0jeB4mwANwkBhru-AgEQgIICjBrKw6tUoxJEzsrctjV3kCK9JBDeGrYKF_19gXFGoopcpe2Kv_UlXGTTND-L3N37Z6Gn_01vUzwzYFzKfyYuesPM> DOI: 10.1603/EC10129
- MALOSHTAN L. M. ET AL: "The study of toxicological properties of "metroxal" cream", vol. 18, no. 2, 10 June 2014 (2014-06-10), pages 14 - 16, XP093071941, ISSN: 1562-725X, Retrieved from the Internet <URL:http://cphj.nuph.edu.ua/article/view/cphj.14.1264/117540> DOI: 10.24959/cphj.14.1264
- SHANG XIAOFEI ET AL: "The toxicity and the acaricidal mechanism against Psoroptes cuniculi of the methanol extract of Adonis coerulea Maxim", VETERINARY PARASITOLOGY, ELSEVIER SCIENCE, AMSTERDAM, NL, vol. 240, 20 April 2017 (2017-04-20), pages 17 - 23, XP085048143, ISSN: 0304-4017, DOI: 10.1016/J.VETPAR.2017.04.019
- TIAN YE ET AL: "Anti-mite Activity and Skin Safety of Herba Taraxaci Extract for Demodex Folliculorum", CHINESE JOURNAL OF PARASITOLOGY AND PARASITIC DISEASES, vol. 25, no. 2, 30 April 2007 (2007-04-30), CN, pages 133 - 136, XP055860104, ISSN: 1000-7423
- "WHO monographs on selected medical plants. Volume 3", 1 January 2007, Switzerland, ISBN: 978-92-4-154702-4, article .: "Radix Gentianae Luteae; Radix Gentianae Scabrae", pages: 150 - 168, XP093072346
- PALIY A. ET AL: "Peculiarities of the course of demodicosis in domestic animals in a megalopolis in the east of Ukraine", REGULATORY MECHANISMS IN BIOSYSTEMS, vol. 14, no. 1, 7 February 2023 (2023-02-07), pages 28 - 33, XP093244547, ISSN: 2519-8521, Retrieved from the Internet <URL:https://medicine.dp.ua/index.php/med/article/download/852/865> DOI: 10.15421/022305
- SHANG XIAOFEI; GUO XIAO; YANG FENG; LI BING; PAN HU; MIAO XIAOLOU; ZHANG JIYU: "The toxicity and the acaricidal mechanism againstPsoroptes cuniculiof the methanol extract ofAdonis coeruleaMaxim", VETERINARY PARASITOLOGY, ELSEVIER SCIENCE, AMSTERDAM., NL, vol. 240, 20 April 2017 (2017-04-20), NL , pages 17 - 23, XP085048143, ISSN: 0304-4017, DOI: 10.1016/j.vetpar.2017.04.019
- FENG BO, ZHU HEYUN GUAN JIAO HAO CHENGYI DUAN JINGCAN GU JINGKAI: "Comparative Study on Pharmacokinetics of Gentiopicroside and Gentianae Radix Extract in Rats", ZHONGYAOCAI - JOURNAL OF CHINESE MEDICINAL MATERIALS, GUOJIA YIYAO GUANLIJU, CN, vol. 36, no. 5, 31 May 2013 (2013-05-31), CN , pages 783 - 786, XP055860103, ISSN: 1001-4454, DOI: 10.13863/j.issn1001-4454.2013.05.028
- TIAN YE, LI CHAOPIN, DENG YUN: "Anti-mite Activity and Skin Safety of Herba Taraxaci Extract for Demodex Folliculorum", CHINESE JOURNAL OF PARASITOLOGY AND PARASITIC DISEASES, ZHONGGUO YUFANG YIXUE KEXUEYUAN JISHENGCHONGBING YANJIUSUO, CN, vol. 25, no. 2, 30 April 2007 (2007-04-30), CN , pages 133 - 136, XP055860104, ISSN: 1000-7423

## Description

### TECHNICAL FIELD

The present invention relates to the field of pharmaceuticals, and in particular taraxerol or taraxasterol acetate for use in preventing and/or treating a disease caused by a *Demodex* spp. infestation. The invention also relates to a non-therapeutic use of taraxerol or taraxasterol acetate for controlling or killing *Demodex* spp.

### BACKGROUND

Mites are a class of tiny animals belonging to Arthropoda, Arachnida, Latigastra, generally having a body size of about 0.5 mm, some as small as 0.1 mm, and most species have a body size of less than 1 mm. It is found that mites are closely related to the health status of human beings and animals (such as dogs, cats and other companion animals). For example, mites such as gamasid mites (*Mesostigmata* spp.), chiggers, itch mite (*Sarcoptes scabiei), Demodex* mites (*Demodex* spp.), flour mites (*Acarus siro*), dust mite and *Pyemotes spp.* are hematophagic and may harm the skin, causing "acne rosacea" or demodicosis, hypersensitivity, urinary acariasis, pulmonary acariasis, intestinal acariasis, scabies and the like, which seriously endanger the health of human beings and animals. *Demodex* mites are microscopic ectoparasites that usually affect the pilosebaceous unit of the skin. Among the reported species, *D. folliculorum* and *D. brevis* have been found on the body surface of humans, and mainly reside in the hair follicles, sebaceous glands and meibomian glands. *Demodex* mites feed on epithelial cells of hair follicle, causing follicle dilatation and hair loss, manifesting clinically as blepharitis marginalis, meibomian gland dysfunction, madarosis, abnormal eyelash alignment, conjunctivitis and blepharoconjunctivitis, pterygium, keratitis, basal cell carcinoma of eyelid and the like. *Demodex* mites may also feed on lipids, causing xerophthalmia. In addition, *Demodex* mites may also cause mechanical obstruction of meibomian gland ducts, causing difficulty in lipid excretion and excessive retention of secretions, leading to the formation of chalazion. The clinical manifestations of the ocular diseases caused by *Demodex* infestation mainly include: recurrent red and itch eyes, dry eyes, burning eyes, foreign body sensation, photophobia and increased secretion; the diseases may also be accompanied by recurrent eyelash loss; blurred vision and decreased vision may occur in severe cases affecting the cornea. In addition to the above ocular diseases, a number of studies in recent years have reported that *Demodex* infestation is associated with a variety of common skin diseases, including seborrhoeic dermatitis, acne, acne rosacea, pityriasis folliculorum, perioral dermatitis, demodicosis, basal cell carcinoma and the like (see, e.g., Luo, X., Li, J., Chen, C., Tseng, S. & Liang, L, Ocular Demodicosis as a Potential Cause of Ocular Surface Inflammation, Cornea 36 Suppl 1, s9-s14; Karincaoglu, Y., Tepe, B., Kalayci, B., Atambay, M. & Seyhan, M, Is Demodex folliculorum an aetiological factor in seborrhoeic dermatitis?, Clinical and experimental dermatology 34, e516-520; Chen, W. & Plewig, G, Human demodicosis: revisit and a proposed classification, The British journal of dermatology 170, 1219-1225).

In particular, reports where the association of mites with ocular diseases has been confirmed include:
Human Permanent Ectoparasites; Recent Advances on Biology and Clinical Significance of Demodex Mites: Narrative Review Article (Iranian journal of parasitology 12(1):12-21) has shown that *D. brevis* lives in the sebaceous glands and meibomian glands and *D. folliculorum* often occupies the hair follicle area of human eyelashes, of which both will cause ocular diseases after infecting the facial skin.
Ocular Demodicosis as a Potential Cause of Ocular Surface Inflammation (*Cornea 36 Suppl 1*(Suppl 1):s9-s14) demonstrates that *D. brevis* and *D. folliculorum* are two *Demodex* species that cause ocular demodicosis in humans, and there is a positive correlation between human age and the risk of the disease and a strong positive correlation between ocular demodicosis and ocular surface inflammatory conditions.
*Demodex* species in human ocular disease: new clinicopathological aspects (International ophthalmology 37(1):303-312) has shown that *D. brevis* and *D. folliculorum* are associated with the pathogenesis of external ocular diseases, and ocular demodicosis can cause an imbalance in the extraocular environment.
Ocular Demodex: a systematic review of the clinical literature (Ophthalmic & physiological optics: the journal of the British College of Ophthalmic Opticians (Optometrists) 40(4):389-432) has shown that *Demodex* infestation is a potential cause of several ocular surface diseases, and the parasitism of *Demodex* mites on the anterior structures of the eyes such as eyelids, eyelashes and the ocular surface can lead to the ocular demodicosis in humans, and the incidence is positively correlated with age.
The relationship between demodex and ocular discomfort (Investigative ophthalmology & visual science 51(6):2906-2911) suggested that the number of *Demodex* mites is positively correlated with the severity of ocular diseases and the age of patients.
*Demodex* mites (Clinics in dermatology 32(6):739-743) suggested that *Demodex* infestation can cause chronic blepharitis marginalis, and the prevalence of chronic blepharitis marginalis is positively correlated with the number of mites and the age of patients.
Quantitative Analysis of the Bacteria in Blepharitis With *Demodex* Infestation (*Frontiers in microbiology* 9:1719) has shown that *D. brevis* and *D. folliculorum* infestations can lead to the occurrence of ocular diseases, the prevalence is positively correlated with the number of mites and the age of patients, and the *Demodex* mites are carriers of *Bacillus spp.,* which can play a role as co-pathogens in the pathogenesis of blepharitis marginalis.
*Bacillus oleronius* and *Demodex* mite infestation in patients with chronic blepharitis (Clinical microbiology and infection: the official publication of the European Society of Clinical Microbiology and Infectious Diseases 18(10):1020-1025) suggested that *Demodex* mites may cause the occurrence of blepharitis marginalis and are carriers of *Bacillus spp.,* which can play a role as co-pathogens in the development of blepharitis marginalis.

Correlation between ocular *Demodex* infestation and serum immunoreactivity to Bacillus proteins in patients with Facial rosacea (Ophthalmology 117(5):870-877.e871) suggested that the incidence of ocular *Demodex* infestation in patients with xerophthalmia is lower than that in patients without xerophthalmia, and that the mite infestation and bacterial infection can coexist in ocular surface inflammation.

High prevalence of demodex brevis infestation in chalazia (American journal of ophthalmology 157(2):342-348.e341) has shown that the incidence of demodicosis caused by *D. brevis* is high in adults and pediatric patients with chalazion, and the ocular demodicosis is a major cause of chalazion.

It is known to treat Demodex folliculorum with ivermectin or 4-terpineol (American Journal of Ophthalmology 2010, 151, 1030-1034; Clinical Optometry 2018, 10, 57-63).

Diseases caused by mites (such as ocular diseases and skin diseases) have not yet received sufficient clinical attention, and are often misdiagnosed as bacterial diseases and the like, while the conventional antibacterial treatments usually have little curative effect.

Therefore, the present invention provides a series of compounds for use in controlling or killing mites.

### SUMMARY

The invention is defined by the appended claims. Namely, the invention is directed to a compound or a salt thereof, or a solvate thereof for use in preventing and/or treating a disease caused by a *Demodex* spp. infestation, by controlling or killing *Demodex* spp. on or in a human or an animal, wherein the compound is taraxerol or taraxasterol acetate. Further, the invention is directed to a non-therapeutic use of a compound or a salt thereof, or a solvate thereof, for controlling or killing *Demodex* spp., wherein the compound is taraxerol or taraxasterol acetate.

Disclosed but not claimed is a use of a compound of formula I or a salt, an isomer or a solvate thereof in preparing a product for controlling or killing mites, wherein the compound of formula I is:

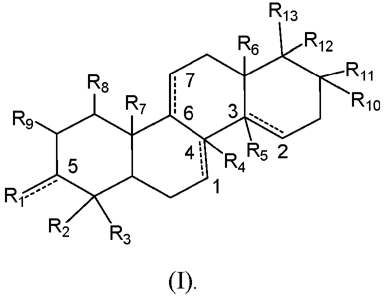

Wherein, R₁ is selected from H, halogen, C₁₋₈ alkyl, O(C₁₋₈ alkyl), S(C₁₋₈ alkyl), NH(C₁₋₈ alkyl), N(C₁₋₈ alkyl)₂, C₃₋₁₁ cycloalkyl, aryl, heteroaryl, C₃₋₁₁ heterocycloalkyl, O(C₁₋₈ cycloalkyl), S(C₁₋₈ cycloalkyl), NH(C₁₋₈ cycloalkyl), N(C₁₋₈ cycloalkyl)(C₁₋₈ alkyl), OH, NH₂, SH, SO₂(C₁₋₈ alkyl), C₂₋₈ alkenyl, C₂₋₈ alkynyl, CH=CH₂, CH=CH(C₁₋₈ alkyl), C(C₁₋₈ alkyl)=CH₂, C(C₁₋₈ alkyl)=CH(C₁₋₈ alkyl), C(C₁₋₈ alkyl)=C(C₁₋₈ alkyl)₂, OC(=O)(C₁₋₈ alkyl), C(=O)(C₁₋₈ alkyl), (C₁₋₈ alkyl)CO₂H, CO₂H, CN, CF₃, CHF₂, CH₂F, NO₂, C(=O)NH(C₁₋₈ alkyl), C(=O)N(C₁₋₈ alkyl)₂, N(C₁₋₈ alkyl)C(=O)NH(C₁₋₈ alkyl), N(C₁₋₈ alkyl)C(=O)N(C₁₋₈ alkyl)₂, NHC(=O)NH(C₁₋₈ alkyl), NHC(=O)N(C₁₋₈ alkyl)₂, NHC(=O)NH₂, N(C₁₋₈ alkyl)SO₂NH(C₁₋₈ alkyl), N(C₁₋₈ alkyl)SO₂N(C₁₋₈ alkyl)₂, NHSO₂NH(C₁₋₈ alkyl) and NHSO₂N(C₁₋₈ alkyl)₂, or when the bond between R₁ and the carbon atom at position 5 is a double bond, R₁ is selected from O and S.

R₂, R₃, R₆, R₇, R₈ and R₉ are independently selected from H, C₁₋₈ alkyl, O(C₁₋₈ alkyl), NH(C₁₋₈ alkyl), N(C₁₋₈ alkyl)₂, C₃₋₁₁ cycloalkyl, O(C₁₋₈ cycloalkyl), OH and NH₂.

R₄ is selected from H, C₁₋₈ alkyl, O(C₁₋₈ alkyl), NH(C₁₋₈ alkyl), N(C₁₋₈ alkyl)₂, C₃₋₁₁ cycloalkyl, O(C₁₋₈ cycloalkyl), OH and NH₂, or when the bond between carbon atoms at positions 1 and 4 is a double bond, R₄ is absent.

R₅ is selected from H, C₁₋₈ alkyl, O(C₁₋₈ alkyl), NH(C₁₋₈ alkyl), N(C₁₋₈ alkyl)₂, C₃₋₁₁ cycloalkyl, O(C₁₋₈ cycloalkyl), OH and NH₂, or when the bond between carbon atoms at positions 2 and 3 is a double bond, R₅ is absent.

The bond between carbon atoms at positions 6 and 7 is a double bond or single bond.

R₁₀ and R₁₃ are independently selected from H, halogen, C₁₋₈ alkyl, O(C₁₋₈ alkyl), S(C₁₋₈ alkyl), NH(C₁₋₈ alkyl), N(C₁₋₈ alkyl)₂, C₃₋₁₁ cycloalkyl, aryl, heteroaryl, C₃₋₁₁ heterocycloalkyl, O(C₁₋₈ cycloalkyl), S(C₁₋₈ cycloalkyl), NH(C₁₋₈ cycloalkyl), N(C₁₋₈ cycloalkyl)(C₁₋₈ alkyl), OH, NH₂, SH, SO₂(C₁₋₈ alkyl), C₂₋₈ alkenyl, C₁₋₈ alkynyl, CH=CH(C₁₋₈ alkyl), CH=CH₂, C(C₁₋₈ alkyl)=CH₂, C(C₁₋₈ alkyl)=CH(C₁₋₈ alkyl), C(C₁₋₈ alkyl)=C(C₁₋₈ alkyl)₂, OC(=O)(C₁₋₈ alkyl), C(=O)(C₁₋₈ alkyl), (C₁₋₈ alkyl)CO₂H, CO₂H, CN, CF₃, CHF₂, CH₂F, NO₂, C(=O)NH(C₁₋₈ alkyl), C(=O)N(C₁₋₈ alkyl)₂, N(C₁₋₈ alkyl)C(=O)NH(C₁₋₈ alkyl), N(C₁₋₈ alkyl)C(=O)N(C₁₋₈ alkyl)₂, NHC(=O)NH(C₁₋₈ alkyl), NHC(=O)N(C₁₋₈ alkyl)₂, NHC(=O)NH₂, N(C₁₋₈ alkyl)SO₂NH(C₁₋₈ alkyl), N(C₁₋₈ alkyl)SO₂N(C₁₋₈ alkyl)₂, NHSO₂NH(C₁₋₈ alkyl) and NHSO₂N(C₁₋₈ alkyl)₂.

R₁₁ and R₁₂ are independently selected from H, halogen, C₁₋₈ alkyl, O(C₁₋₈ alkyl), S(C₁₋₈ alkyl), NH(C₁₋₈ alkyl), N(C₁₋₈ alkyl)₂, C₃₋₁₁ cycloalkyl, aryl, heteroaryl, C₃₋₁₁ heterocycloalkyl, O(C₁₋₈ cycloalkyl), S(C₁₋₈ cycloalkyl), NH(C₁₋₈ cycloalkyl), N(C₁₋₈ cycloalkyl)(C₁₋₈ alkyl), OH, NH₂, SH, SO₂(C₁₋₈ alkyl), C₂₋₈ alkenyl, C₁₋₈ alkynyl, CH=CH(C₁₋₈ alkyl), C(C₁₋₈ alkyl)=CH(C₁₋₈ alkyl), C(C₁₋₈ alkyl)=C(C₁₋₈ alkyl)₂, OC(=O)(C₁₋₈ alkyl), C(=O)(C₁₋₈ alkyl), (C₁₋₈ alkyl)CO₂H, CO₂H, CN, CF₃, CHF₂, CH₂F, NO₂, C(=O)NH(C₁₋₈ alkyl), C(=O)N(C₁₋₈ alkyl)₂, N(C₁₋₈ alkyl)C(=O)NH(C₁₋₈ alkyl), N(C₁₋₈ alkyl)C(=O)N(C₁₋₈ alkyl)₂, NHC(=O)NH(C₁₋₈ alkyl), NHC(=O)N(C₁₋₈ alkyl)₂, NHC(=O)NH₂, N(C₁₋₈ alkyl)SO₂NH(C₁₋₈ alkyl), N(C₁₋₈ alkyl)SO₂N(C₁₋₈ alkyl)₂, NHSO₂NH(C₁₋₈ alkyl) and NHSO₂N(C₁₋₈ alkyl)₂, or, R₁₁ and R₁₂ are carbon atoms and together with carbon atoms therebetween form a 5- to 8-membered ring, wherein H on any carbon atom on the ring may be substituted with C₁₋₈ alkyl, O(C₁₋₈ alkyl), NH(C₁₋₈ alkyl), N(C₁₋₈ alkyl)₂, C₃₋₁₁ cycloalkyl, O(C₁₋₈ cycloalkyl), OH, NH₂, C₂₋₈ alkenyl, C₂₋₈ alkynyl, CH=CH₂, C(C₁₋₈ alkyl)=CH₂, CH=CH(C₁₋₈ alkyl), C(C₁₋₈ alkyl)=CH(C₁₋₈ alkyl) or C(C₁₋₈ alkyl)=C(C₁₋₈ alkyl)₂.

Specifically, the compound of formula I described herein is a compound of formula I-1:

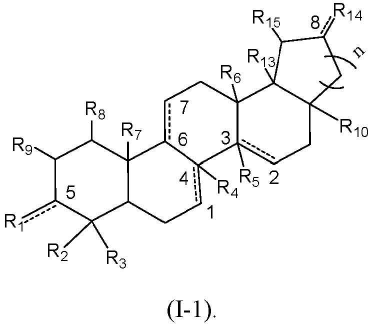

Wherein, when the bond between R₁₄ and the carbon atom at position 8 is a single bond, R₁₄ is selected from H, halogen, C₁₋₈ alkyl, O(C₁₋₈ alkyl), S(C₁₋₈ alkyl), NH(C₁₋₈ alkyl), N(C₁₋₈ alkyl)₂, C₃₋₁₁ cycloalkyl, aryl, heteroaryl, C₃₋₁₁ heterocycloalkyl, O(C₁₋₈ cycloalkyl), S(C₁₋₈ cycloalkyl), NH(C₁₋₈ cycloalkyl), N(C₁₋₈ cycloalkyl)(C₁₋₈ alkyl), OH, NH₂, SH, SO₂(C₁₋₈ alkyl), C₂₋₈ alkenyl, C₂₋₈ alkynyl, CH=CH(C₁₋₈ alkyl), C(C₁₋₈ alkyl)=CH(C₁₋₈ alkyl), C(C₁₋₈ alkyl)=C(C₁₋₈ alkyl)₂, OC(=O)(C₁₋₈ alkyl), C(=O)(C₁₋₈ alkyl), (C₁₋₈ alkyl)CO₂H, CO₂H, CN, CF₃, CHF₂, CH₂F, NO₂, C(=O)NH(C₁₋₈ alkyl), C(=O)N(C₁₋₈ alkyl)₂, N(C₁₋₈ alkyl)C(=O)NH(C₁₋₈ alkyl), N(C₁₋₈ alkyl)C(=O)N(C₁₋₈ alkyl)₂, NHC(=O)NH(C₁₋₈ alkyl), NHC(=O)N(C₁₋₈ alkyl)₂, NHC(=O)NH₂, N(C₁₋₈ alkyl)SO₂NH(C₁₋₈ alkyl), N(C₁₋₈ alkyl)SO₂N(C₁₋₈ alkyl)₂, NHSO₂NH(C₁₋₈ alkyl) and NHSO₂N(C₁₋₈ alkyl)₂, and hydrogen atom on the carbon atom at position 8 is optionally substituted with R₁₄; when the bond between R₁₄ and the carbon atom on position 8 is a double bond, R₁₄ is selected from O, S, CH₂, CH(C₁₋₈ alkyl) and C(C₁₋₈ alkyl)₂.

R₁₅ is selected from H, halogen, C₁₋₈ alkyl, O(C₁₋₈ alkyl), S(C₁₋₈ alkyl), NH(C₁₋₈ alkyl), N(C₁₋₈ alkyl)₂, C₃₋₁₁ cycloalkyl, aryl, heteroaryl, C₃₋₁₁ heterocycloalkyl, O(C₁₋₈ cycloalkyl), S(C₁₋₈ cycloalkyl), NH(C₁₋₈ cycloalkyl), N(C₁₋₈ cycloalkyl)(C₁₋₈ alkyl), OH, NH₂, SH, SO₂(C₁₋₈ alkyl), C₂₋₈ alkenyl, C₂₋₈ alkynyl, CH=CH(C₁₋₈ alkyl), C(C₁₋₈ alkyl)=CH(C₁₋₈ alkyl), C(C₁₋₈ alkyl)=C(C₁₋₈ alkyl)₂, OC(=O)(C₁₋₈ alkyl), C(=O)(C₁₋₈ alkyl), (C₁₋₈ alkyl)CO₂H, CO₂H, CN, CF₃, CHF₂, CH₂F, NO₂, C(=O)NH(C₁₋₈ alkyl), C(=O)N(C₁₋₈ alkyl)₂, N(C₁₋₈ alkyl)C(=O)NH(C₁₋₈ alkyl), N(C₁₋₈ alkyl)C(=O)N(C₁₋₈ alkyl)₂, NHC(=O)NH(C₁₋₈ alkyl), NHC(=O)N(C₁₋₈ alkyl)₂, NHC(=O)NH₂, N(C₁₋₈ alkyl)SO₂NH(C₁₋₈ alkyl), N(C₁₋₈ alkyl)SO₂N(C₁₋₈ alkyl)₂, NHSO₂NH(C₁₋₈ alkyl) and NHSO₂N(C₁₋₈ alkyl)₂.

n is an integer of 1-4, such as 1, 2, 3 or 4.

Specifically, R₁ is selected from H, C₁₋₃ alkyl, OH, OC(=O)(C₁₋₃ alkyl), (C₁₋₃ alkyl)CO₂H, CF₃, CHF₂ and CH₂F, or when the bond between R₁ and the carbon atom at position 5 is a double bond, R₁ is O. More specifically, R₁ is selected from OH and OC(=O)(C₁₋₃ alkyl). Still more specifically, R₁ is selected from OH and OC(=O)CH₃.

Specifically, R₂, R₃, R₆, R₇, R₈ and R₉ are independently selected from H, C₁-₃ alkyl, O(C₁₋₃ alkyl), C₃₋₆ cycloalkyl, OH and NH₂. Still more specifically, R₂, R₃, R₆, R₇, R₈ and R₉ are independently selected from H and methyl.

Specifically, the bond between carbon atoms at positions 1 and 4 is a single bond, and R₄ is selected from H, C₁₋₃ alkyl, O(C₁₋₃ alkyl), C₃₋₆ cycloalkyl, O(C₁₋₃ cycloalkyl) and OH. More specifically, the bond between carbon atoms at positions 1 and 4 is a single bond, and R₄ is selected from H and methyl.

Specifically, the bond between carbon atoms at positions 2 and 3 is a single bond, and R₅ is selected from H, C₁₋₃ alkyl, O(C₁₋₃ alkyl), C₃₋₆ cycloalkyl, O(C₁₋₃ cycloalkyl) and OH. More specifically, the bond between carbon atoms at positions 2 and 3 is a single bond, and R₅ is selected from H and methyl.

Specifically, the bond between carbon atoms at positions 6 and 7 is a single bond.

Specifically, R₁₀ and R₁₃ are independently selected from H, C₁₋₃ alkyl, O(C₁₋₃ alkyl), C₃₋₆ cycloalkyl, OH and NH₂. More specifically, R₁₀ and R₁₃ are independently selected from H and methyl.

Specifically, when the bond between R₁₄ and the carbon atom at position 8 is a single bond, R₁₄ is selected from H, C₁₋₃ alkyl, O(C₁₋₃ alkyl), C₃₋₆ cycloalkyl, OH and NH₂, and similarly, the hydrogen atom on the carbon atom at position 8 is substituted with R₁₄; when the bond between R₁₄ and the carbon atom at position 8 is a double bond, R₁₄ is selected from CH₂, CH(C₁₋₃ alkyl) and C(C₁₋₃ alkyl)₂. More specifically, when the bond between R₁₄ and the carbon atom at position 8 is a single bond, R₁₄ is selected from H and methyl, and similarly, the hydrogen atom on the carbon atom at position 8 is substituted with H or methyl; when the bond between R₁₄ and the carbon atom at position 8 is a double bond, R₁₄ is CH₂ (methylene).

Specifically, R₁₅ is selected from H, C₁₋₃ alkyl, O(C₁₋₃ alkyl), C₃₋₆ cycloalkyl, OH, NH₂, CH=CH(C₁₋₃ alkyl), C(C₁₋₃ alkyl)=CH₂, C(C₁₋₃ alkyl)=CH(C₁₋₃ alkyl) and C(C₁₋₃ alkyl)=C(C₁₋₃ alkyl)₂. More specifically, R₁₅ is selected from H, C₁₋₃ alkyl and C(CH₃)=CH₂.

More specifically, the compound of formula I is taraxasterol, taraxerol, taraxerone, roburic acid, taraxasterol acetate, taraxeryl acetate, lupenone, or a salt, an isomer or a solvate thereof.

According to the claimed invention, taraxerol or taraxasterol acetate, or a salt or solvate thereof are used.

Further disclosed but not claimed is the use of a compound of formula II or a salt, an isomer or a solvate thereof in preparing a product for controlling or killing mites, wherein the compound of formula II is:

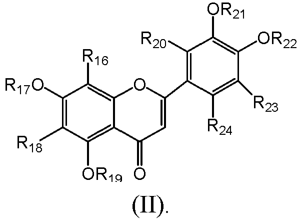

Wherein, R₁₆, R₁₈, R₂₀, R₂₃ and R₂₄ are independently selected from H, halogen, C₁₋₈ alkyl, O(C₁₋₈ alkyl), S(C₁₋₈ alkyl), NH(C₁₋₈ alkyl), N(C₁₋₈ alkyl)₂, C₃₋₁₁ cycloalkyl, aryl, heteroaryl, C₃₋₁₁ heterocycloalkyl, O(C₁₋₈ cycloalkyl), S(C₁₋₈ cycloalkyl), NH(C₁₋₈ cycloalkyl), N(C₁₋₈ cycloalkyl)(C₁₋₈ alkyl), OH, NH₂, SH, SO₂(C₁₋₈ alkyl), C₂₋₈ alkenyl, C₂₋₈ alkynyl, CH=CH(C₁₋₈ alkyl), C(C₁₋₈ alkyl)=CH(C₁₋₈ alkyl), C(C₁₋₈ alkyl)=C(C₁₋₈ alkyl)₂, OC(=O)(C₁₋₈ alkyl), C(=O)(C₁₋₈ alkyl), (C₁₋₈ alkyl)CO₂H, CO₂H, CN, CF₃, CHF₂, CH₂F, NO₂, C(=O)NH(C₁₋₈ alkyl), C(=O)N(C₁₋₈ alkyl)₂, N(C₁₋₈ alkyl)C(=O)NH(C₁₋₈ alkyl), N(C₁₋₈ alkyl)C(=O)N(C₁₋₈ alkyl)₂, NHC(=O)NH(C₁₋₈ alkyl), NHC(=O)N(C₁₋₈ alkyl)₂, NHC(=O)NH₂, N(C₁₋₈ alkyl)SO₂NH(C₁₋₈ alkyl), N(C₁₋₈ alkyl)SO₂N(C₁₋₈ alkyl)₂, NHSO₂NH(C₁₋₈ alkyl) and NHSO₂N(C₁₋₈ alkyl)₂.

R₁₇, R₁₉, R₂₁ and R₂₂ are independently selected from H, C₁₋₈ alkyl, O(C₁₋₈ alkyl), C₃₋₁₁ cycloalkyl, aryl, heteroaryl, C₃₋₁₁ heterocycloalkyl, O(C₁₋₈ cycloalkyl) and a group of formula II-1, II-2 or II-3:

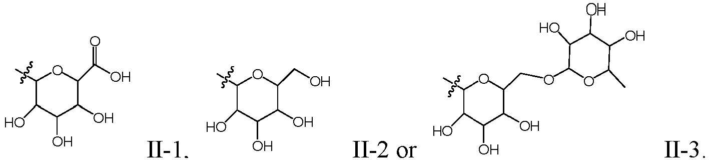

Specifically, R₁₆, R₁₈, R₂₀, R₂₃ and R₂₄ are independently selected from H, halogen, C₁₋₃ alkyl, O(C₁₋₃ alkyl), NH(C₁₋₃ alkyl), N(C₁₋₃ alkyl)₂, C₃₋₁₁ cycloalkyl, OH, NH₂, OC(=O)(C₁₋₃ alkyl), C(=O)(C₁₋₃ alkyl), (C₁₋₃ alkyl)CO₂H, CO₂H, CN, CF₃, CHF₂, CH₂F, NO₂, C₂₋₃ alkenyl, C₂₋₃ alkynyl, CH=CH(C₁₋₃ alkyl), C(C₁₋₃ alkyl)=CH(C₁₋₃ alkyl) and C(C₁₋₃ alkyl)=C(C₁₋₃ alkyl)₂. More specifically, R₁₆, R₁₈, R₂₀, R₂₃ and R₂₄ are independently selected from H and methyl.

Specifically, R₁₇, R₁₉, R₂₁ and R₂₂ are independently selected from H, C₁₋₃ alkyl, O(C₁₋₃ alkyl) and a group of formula II-1 or II-2. More specifically, R₁₇, R₁₉, R₂₁ and R₂₂ are independently selected from H and methyl.

In an embodiment not according to the claimed invention, the compound of formula II is diosmetin or a salt, an isomer or a solvate thereof.

Further disclosed but not claimed is the use of a compound of formula III or a salt, an isomer or a solvate thereof in preparing a product for controlling or killing mites, wherein the compound of formula III is:

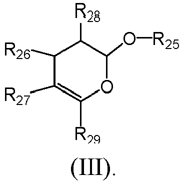

Wherein, R₂₅ is selected from 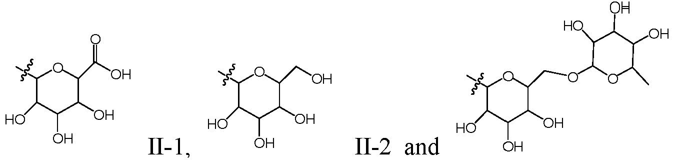 II-3.

R₂₆ is a carbon atom and together with at least one of R₂₇ and R₂₈ forms a 5- to 8-membered ring, and the one of R₂₇ and R₂₈ that does not form the ring with R₂₆ is selected from H, halogen, C₁₋₈ alkyl, O(C₁₋₈ alkyl), S(C₁₋₈ alkyl), NH(C₁₋₈ alkyl), N(C₁₋₈ alkyl)₂, C₃₋₁₁ cycloalkyl, aryl, heteroaryl, C₃₋₁₁ heterocycloalkyl, O(C₁₋₈ cycloalkyl), S(C₁₋₈ cycloalkyl), NH(C₁₋₈ cycloalkyl), N(C₁₋₈ cycloalkyl)(C₁₋₈ alkyl), OH, NH₂, SH, SO₂(C₁₋₈ alkyl), C₂₋₈ alkenyl, C₂₋₈ alkynyl, CH=CH(C₁₋₈ alkyl), C(C₁₋₈ alkyl)=CH(C₁₋₈ alkyl), C(C₁₋₈ alkyl)=C(C₁₋₈ alkyl)₂, OC(=O)(C₁₋₈ alkyl), C(=O)(C₁₋₈ alkyl), (C₁₋₈ alkyl)CO₂H, CO₂H, CN, CF₃, CHF₂, CH₂F, NO₂, C(=O)NH(C₁₋₈ alkyl), C(=O)N(C₁₋₈ alkyl)₂, N(C₁₋₈ alkyl)C(=O)NH(C₁₋₈ alkyl), N(C₁₋₈ alkyl)C(=O)N(C₁₋₈ alkyl)₂, NHC(=O)NH(C₁₋₈ alkyl), NHC(=O)N(C₁₋₈ alkyl)₂, NHC(=O)NH₂, N(C₁₋₈ alkyl)SO₂NH(C₁₋₈ alkyl), N(C₁₋₈ alkyl)SO₂N(C₁₋₈ alkyl)₂, NHSO₂NH(C₁₋₈ alkyl) and NHSO₂N(C₁₋₈ alkyl)₂.

R₂₉ is selected from H, halogen, C₁₋₈ alkyl, O(C₁₋₈ alkyl), S(C₁₋₈ alkyl), NH(C₁₋₈ alkyl), N(C₁₋₈ alkyl)₂, C₃₋₁₁ cycloalkyl, aryl, heteroaryl, C₃₋₁₁ heterocycloalkyl, O(C₁₋₈ cycloalkyl), S(C₁₋₈ cycloalkyl), NH(C₁₋₈ cycloalkyl), N(C₁₋₈ cycloalkyl)(C₁₋₈ alkyl), OH, NH₂, SH, SO₂(C₁₋₈ alkyl), C₂₋₈ alkenyl, C₂₋₈ alkynyl, CH=CH(C₁₋₈ alkyl), C(C₁₋₈ alkyl)=CH(C₁₋₈ alkyl), C(C₁₋₈ alkyl)=C(C₁₋₈ alkyl)₂, OC(=O)(C₁₋₈ alkyl), C(=O)(C₁₋₈ alkyl), (C₁₋₈ alkyl)CO₂H, CO₂H, CN, CF₃, CHF₂, CH₂F, NO₂, C(=O)NH(C₁₋₈ alkyl), C(=O)N(C₁₋₈ alkyl)₂, N(C₁₋₈ alkyl)C(=O)NH(C₁₋₈ alkyl), N(C₁₋₈ alkyl)C(=O)N(C₁₋₈ alkyl)₂, NHC(=O)NH(C₁₋₈ alkyl), NHC(=O)N(C₁₋₈ alkyl)₂, NHC(=O)NH₂, N(C₁₋₈ alkyl)SO₂NH(C₁₋₈ alkyl), N(C₁₋₈ alkyl)SO₂N(C₁₋₈ alkyl)₂, NHSO₂NH(C₁₋₈ alkyl) and NHSO₂N(C₁₋₈ alkyl)₂.

Specifically, the compound of formula III described herein is a compound of formula III-1 or III-2:

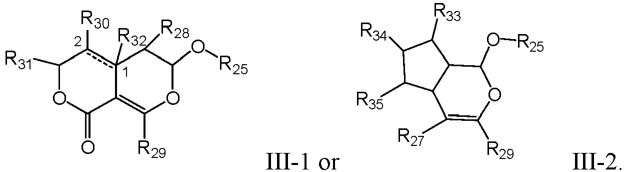

Wherein, R₃₀, R₃₁, R₃₃, R₃₄ and R₃₅ are independently selected from H, halogen, C₁₋₈ alkyl, O(C₁₋₈ alkyl), S(C₁₋₈ alkyl), NH(C₁₋₈ alkyl), N(C₁₋₈ alkyl)₂, C₃₋₁₁ cycloalkyl, aryl, heteroaryl, C₃₋₁₁ heterocycloalkyl, O(C₁₋₈ cycloalkyl), S(C₁₋₈ cycloalkyl), NH(C₁₋₈ cycloalkyl), N(C₁₋₈ cycloalkyl)(C₁₋₈ alkyl), OH, NH₂, SH, SO₂(C₁₋₈ alkyl), C₂₋₈ alkenyl, C₂₋₈ alkynyl, CH=CH(C₁₋₈ alkyl), C(C₁₋₈ alkyl)=CH(C₁₋₈ alkyl), C(C₁₋₈ alkyl)=C(C₁₋₈ alkyl)₂, OC(=O)(C₁₋₈ alkyl), C(=O)(C₁₋₈ alkyl), (C₁₋₈ alkyl)CO₂H, CO₂H, CN, CF₃, CHF₂, CH₂F, NO₂, C(=O)NH(C₁₋₈ alkyl), C(=O)N(C₁₋₈ alkyl)₂, N(C₁₋₈ alkyl)C(=O)NH(C₁₋₈ alkyl), N(C₁₋₈ alkyl)C(=O)N(C₁₋₈ alkyl)₂, NHC(=O)NH(C₁₋₈ alkyl), NHC(=O)N(C₁₋₈ alkyl)₂, NHC(=O)NH₂, N(C₁₋₈ alkyl)SO₂NH(C₁₋₈ alkyl), N(C₁₋₈ alkyl)SO₂N(C₁₋₈ alkyl)₂, NHSO₂NH(C₁₋₈ alkyl) and NHSO₂N(C₁₋₈ alkyl)₂.

In formula III-1, the bond between carbon atom at positions 1 and 2 is selected from a double bond and a single bond. When in formula III-1 the bond between the carbon atoms at positions 1 and 2 is a double bond, R₃₂ is absent; when in formula III-1 the bond between the carbon atoms at positions 1 and 2 is a single bond, R₃₂ is selected from H, halogen, C₁₋₈ alkyl, O(C₁₋₈ alkyl), S(C₁₋₈ alkyl), NH(C₁₋₈ alkyl), N(C₁₋₈ alkyl)₂, C₃₋₁₁ cycloalkyl, aryl, heteroaryl, C₃₋₁₁ heterocycloalkyl, O(C₁₋₈ cycloalkyl), S(C₁₋₈ cycloalkyl), NH(C₁₋₈ cycloalkyl), N(C₁₋₈ cycloalkyl)(C₁₋₈ alkyl), OH, NH₂, SH, SO₂(C₁₋₈ alkyl), C₂₋₈ alkenyl, C₂₋₈ alkynyl, CH=CH(C₁₋₈ alkyl), C(C₁₋₈ alkyl)=CH(C₁₋₈ alkyl), C(C₁₋₈ alkyl)=C(C₁₋₈ alkyl)₂, OC(=O)(C₁₋₈ alkyl), C(=O)(C₁₋₈ alkyl), (C₁₋₈ alkyl)CO₂H, CO₂H, CN, CF₃, CHF₂, CH₂F, NO₂, C(=O)NH(C₁₋₈ alkyl), C(=O)N(C₁₋₈ alkyl)₂, N(C₁₋₈ alkyl)C(=O)NH(C₁₋₈ alkyl), N(C₁₋₈ alkyl)C(=O)N(C₁₋₈ alkyl)₂, NHC(=O)NH(C₁₋₈ alkyl), NHC(=O)N(C₁₋₈ alkyl)₂, NHC(=O)NH₂, N(C₁₋₈ alkyl)SO₂NH(C₁₋₈ alkyl), N(C₁₋₈ alkyl)SO₂N(C₁₋₈ alkyl)₂, NHSO₂NH(C₁₋₈ alkyl) and NHSO₂N(C₁₋₈ alkyl)₂.

Specifically, R₂₅ is

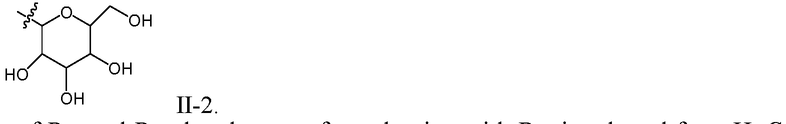

Specifically, the one of R₂₇ and R₂₈ that does not form the nng with R₂₆ is selected from H, C₁₋₃ alkyl, O(C₁₋₃ alkyl), O(C₁₋₃ cycloalkyl), C₂₋₃ alkenyl, C₂₋₃ alkynyl, CH=CH(C₁₋₃ alkyl), C(C₁₋₃ alkyl)=CH(C₁₋₃ alkyl), C(C₁₋₃ alkyl)=C(C₁₋₃ alkyl)₂, OC(=O)(C₁₋₃ alkyl), C(=O)(C₁₋₃ alkyl), (C₁₋₃ alkyl)CO₂H and CO₂H. More specifically, the one of R₂₇ and R₂₈ that does not form the ring with R₂₆ is selected from H, CH=CH₂ and CO₂H.

Specifically, R₃₀, R₃₁, R₃₃, R₃₄ and R₃₅ are independently selected from H, C₁₋₃ alkyl, O(C₁₋₃ alkyl), C₃₋₆ cycloalkyl, OH, NH₂, CH=CH(C₁₋₃ alkyl), C(C₁₋₃ alkyl)=CH₂, C(C₁₋₃ alkyl)=CH(C₁₋₃ alkyl) and C(C₁₋₃ alkyl)=C(C₁₋₃ alkyl)₂. More specifically, R₃₀, R₃₁, R₃₃, R₃₄ and R₃₅ are independently selected from H, methyl and OH.

Specifically, when the bond between carbon atom at positions 1 and 2 in formula III-1 is a double bond, R₃₂ is absent.

In an embodiment not according to the claimed invention, the compound of formula III is swertiamarin, sweroside, gentiopicroside, loganic acid or a salt, an isomer or a solvate thereof.

Specifically, the compound or the salt, the isomer or the solvate thereof described herein can be used as the sole active ingredient for controlling or killing mites, and can also be used in combination with other ingredients with the same or different activities for controlling or killing mites.

The compounds described herein can be obtained by extraction from natural plants, such as Herba Taraxaci, *Gentiana macrophylla,* gentian, Chrysanthemum and the like, and the extraction method can be a conventional method in the art.

The compounds described herein can also be prepared by chemical synthesis or biosynthesis. Specifically, the product for killing mites described above can be used for therapeutic and/or prophylactic purposes, and also for non-therapeutic and/or non-prophylactic purposes.

Specifically, the mites described above can be one or more of *Demodex spp., Dermatophagoides pteronyssinus, Sarcoptes scabiei.* and the like. According to the present invention, the mites described above are *Demodex spp.,* such as *D. folliculorum* and *D. brevis.*

In an embodiment of the present invention, the product for killing mites described above is a pharmaceutical composition.

Specifically, the pharmaceutical composition described above further comprises a pharmaceutically acceptable excipient(s).

Specifically, the pharmaceutical composition described above is used for preventing and/or treating a disease caused by mite infestation.

Specifically, the disease described above may be an ocular disease, a skin disease, an allergic disease and the like.

Specifically, the ocular disease described above may be one or more of blepharitis marginalis, meibomian gland dysfunction, tarsitis, madarosis, abnormal eyelash alignment, glaucoma, cataract, ocular folliculitis, conjunctivitis, blepharoconjunctivitis, pterygium, keratitis, eyelid laxity and ectropion, basal cell carcinoma of eyelid, xerophthalmia, chalazion and the like; the ocular disease may have one or more symptoms selected from: red and itchy eyes, dry eyes, burning eyes, foreign body sensation, photophobia, increased eye discharge, loss of eyelashes, blurred vision, decreased vision and the like.

Specifically, the skin disease described above may be one or more of seborrhoeic dermatitis, acne, acne rosacea, pityriasis folliculorum, perioral dermatitis, demodicosis, gaile, basal cell carcinoma and the like.

Specifically, the allergic disease described above may be one or more of allergic asthma, allergic rhinitis, allergic dermatitis and the like.

Specifically, the pharmaceutical composition described above may be in any dosage form suitable for administration, such as a topical preparation, in particular, an ophthalmic preparation, a skin topical preparation and the like.

Specifically, the ophthalmic preparation described above may be an eye drop, an eye ointment, an ophthalmic gel, an ophthalmic emulsion, an ophthalmic suspension, an ophthalmic film, a collyrium, an intraocular injection and the like.

Specifically, the ophthalmic preparation may comprise a pharmaceutically acceptable excipient(s) such as a pH regulator, a co-solvent, an osmotic pressure regulator, a viscosity regulator, an antioxidant, a bacteriostatic preservative, a buffer, a suspending agent, a local anesthetic, a surfactant, a solubilizer, a wetting agent, an emulsifier, a stabilizer, a filler, a protective agent, a solvent and the like.

Specifically, the skin topical preparation described above may be an aerosol, a powder, a lotion, a tincture, a liniment, a film, an ointment, a gel, a paste, an emulsion and the like.

Specifically, the dosage forms of the pharmaceutical composition described above can be prepared according to conventional production methods in the field of pharmaceuticals.

Specifically, the pharmaceutical composition described above may comprise 0.01%-99.5% (e.g., 0.01%, 0.1%, 0.5%, 1%, 2%, 3%, 4%, 5%, 6%, 7%, 8%, 9%, 10%, 20%, 30%, 40%, 50%, 60%, 70%, 80%, 90%, 95%, 99% or 99.5%) by weight of the active ingredient.

Specifically, the pharmaceutical composition described above can be used in humans, and can also be used as a veterinary drug in non-human animals, such as non-human mammals, e.g., companion animals (e.g., dogs, cats, rabbits, mice, etc.), livestock animals (e.g., horses, cows, sheep, pigs, dogs, rabbits, etc.) and the like.

In another embodiment of the present invention, the product for killing mites described above is a cosmetic product.

Specifically, the cosmetic product described above further comprises a cosmetically acceptable excipient(s).

Specifically, the cosmetic product described above may be a cosmetic product used for the face, such as a facial cleanser, a soap, a skin softener, a toner, a skin care lotion, a jelly lotion, a facial cream, a sunscreen cream, an essence, a facial mask, a gel, a foundation and a scrub.

Specifically, the cosmetic product described above may be a cosmetic product used for parts of the body other than the face, such as a neck cream, a shampoo, a shower gel, a hair conditioner, a body lotion, a scrub and the like.

Specifically, the cosmetic product described above may also be a cosmetic product used for the eyes and periocular region, such as an eye cream, a mascara, an eyeliner powder, a cream eyeliner, an eyeliner pencil, an eye shadow powder, an eye shadow cream, an eyebrow pencil, a brow powder and the like.

Specifically, the various forms of the cosmetic product described above can be prepared according to conventional production methods in the field of cosmetics.

Specifically, the cosmetic product described above may comprise 0.01%-99.5% (e.g., 0.01%, 0.1%, 0.5%, 1%, 2%, 3%, 4%, 5%, 6%, 7%, 8%, 9%, 10%, 20%, 30%, 40%, 50%, 60%, 70%, 80%, 90%, 95%, 99% or 99.5%) by weight of the active ingredient.

In another embodiment of the present invention, the product for killing mites described above is an acaricide which can be used non-therapeutically for killing and controlling mites that may live in articles in the living environment (e.g., pillow case, pillow inner, bed sheet, bedding, mattress, clothing, carpet, cushion, sofa, summer sleeping mat, plush toy, air conditioner and the like).

Specifically, the acaricide described above may comprise any suitable excipient that can achieve the desired properties.

Specifically, the acaricide described above may be in a form of a spray, a lotion, a paster, a small packet and the like.

Specifically, the various forms of the acaricide described above can be prepared according to conventional production methods in the field of daily care products.

Specifically, the acaricide described above may comprise 0.01%-99.5% (e.g., 0.01%, 0.1%, 0.5%, 1%, 2%, 3%, 4%, 5%, 6%, 7%, 8%, 9%, 10%, 20%, 30%, 40%, 50%, 60%, 70%, 80%, 90%, 95%, 99% or 99.5%) by weight of the active ingredient.

The present invention further provides taraxerol or taraxasterol acteate for use in preventing and/or treating a disease caused by a *Demodex* spp. infestation, comprising administering to a human or animal in need thereof the compound or the salt, or the solvate thereof.

Specifically, the disease described above may be an ocular disease, a skin disease, or an allergic disease.

Specifically, the ocular disease described above may be one or more of blepharitis marginalis, meibomian gland dysfunction, tarsitis, madarosis, abnormal eyelash alignment, glaucoma, cataract, ocular folliculitis, conjunctivitis, blepharoconjunctivitis, pterygium, keratitis, eyelid laxity and ectropion, basal cell carcinoma of eyelid, xerophthalmia, chalazion and the like; the ocular disease may have one or more symptoms selected from: red and itchy eyes, dry eyes, burning eyes, foreign body sensation, photophobia, increased eye discharge, loss of eyelashes, blurred vision, or decreased vision.

Specifically, the skin disease described above may be one or more of seborrhoeic dermatitis, acne, acne rosacea, pityriasis folliculorum, perioral dermatitis, demodicosis, gaile, or basal cell carcinoma.

Specifically, the allergic disease described above may be one or more of allergic asthma, allergic rhinitis, or allergic dermatitis.

Specifically, the subject described above can be any animal receiving the prophylaxis and/or treatment, in particular, a mammal, such as a human, a cat, a dog, a rabbit, a mouse, a horse, a cow, a sheep, a pig and the like. In an embodiment of the present invention, the subject described above is a human; and in another embodiment of the present invention, the subject described above is a non-human animal.

In an embodiment of the present invention, the compound for use described above is a compound for use in treating and/or preventing an ocular disease caused by mites, comprising administering to eyes of a subject in need thereof the compound or the salt, or the solvate thereof described above. According to the claimed invention the compound for use is taraxerol or taraxasterol acetate.

Specifically, the ophthalmic administration described above may be an administration in a form of an eye drop, an eye ointment, an ophthalmic gel, an ophthalmic emulsion, an ophthalmic suspension, an ophthalmic film, a collyrium or an intraocular injection.

Specifically, the dose of the compounds or the pharmaceutical composition described above may vary according to the route of administration, the age and body weight of the subject, the disease to be treated in the subject and the severity, etc., and may be administered in one or more doses.

It is found by comparison that the compounds described herein can reduce the survival time of mites as compared with the control group. In particular, taraxerol, diosmetin, gentiopicroside and taraxasterol acetate can significantly shorten the survival time of mites, and can be used for preparing a product (such as a drug, a cosmetic product, a daily product and the like) for killing or controlling mites. According to the invention taraxerol or taraxasterol acetate are used for killing or controlling mites.

### DETAILED DESCRIPTION

Unless otherwise defined, all scientific and technical terms used herein have the same meaning as commonly understood by those skilled in the art to which the present invention relates.

In the present invention, the term "animal" generally refers to vertebrates, in particular, mammals, including humans. The term "non-human animal" refers to any vertebrate except human beings, in particular, mammals. In some embodiments of the present invention, the non-human animal described herein is a domestic animal, i.e., an animal raised and domesticated by humans with artificially controlled reproduction for purposes such as food, labor, fur, companionship and experiment, such as an economic animal, a companion animal and a laboratory animal. The economic animal may be, for example, a livestock animal such as a pig, a cow, a sheep, a horse, a donkey, a fox, a raccoon dog, a mink, a camel and the like. The companion animal may be, for example, a dog, a cat, a rabbit, a murine (such as a guinea pig, a hamster, a gerbil, a chinchilla, a squirrel, etc.) and the like. The laboratory animal may be, for example, a monkey, a dog, a rabbit, a cat, a murine (such as a rat and a mouse) and the like.

The technical schemes of the present invention will be clearly and completely described below with reference to the examples of the present invention.

### Example 1: Preliminary screening in vitro of compounds for effect on survival time of Demodex mites

### Methodology:

### 1. Isolated compound

The following isolated compounds were selected. The names and molecular formulas are shown in Table 1 below.

The isolated compounds were purchased from Chengdu Herbpurify Co., Ltd., as standard references.

According to the invention taraxerol and taraxasterol acetate are used. Any other compounds are described for reference only.

**Table 1: Names and molecular formulas of isolated compounds**

| No. | Chinese name | English name | Molecular formula |
|---|---|---|---|
| 1 | 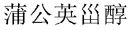 | Taraxasterol | C30H50O |
| 2 | 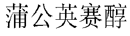 | Taraxerol | C30H50O |
| 3 | 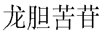 | Gentiopicroside | C16H20O9 |
| 4 | 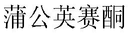 | Taraxerone | C30H48O |
| 5 | 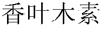 | Diosmetin | C16H12O6 |
| 6 | 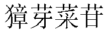 | Sweroside | C16H22O9 |
| 7 | 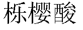 | Roburic acid | C30H48O2 |
| 8 | 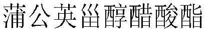 | Taraxasterol acetate | C32H52O2 |
| 9 | 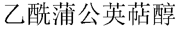 | Taraxeryl acetate | C32H52O2 |
| 10 | 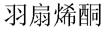 | Lupenone | C30H48O |
| 11 | 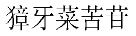 | Swertiamarine | C16H22O10 |
| 12 | 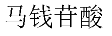 | Loganic acid | C16H24O10 |

### 2. Preparation of solutions

The powders of the reference compounds shown in Table 1 were separately dissolved in dimethyl sulfoxide (DMSO), and then diluted with sterilized double-distilled water to make the final concentration of DMSO 10%, which could not shorten the survival time of Demodex mites as per a controlled study. The negative control group received DMSO mixed with sterilized water at a final concentration of 10%.

### 3. In-vitro culture of Demodex mites

35 µL of each solution was added to a glass slide, and the survival of the mites was observed under an optical microscope every 2 hours. During the experiment, the *Demodex* mites were observed by two skilled operators separately for the activities (bodies, limbs and the like) through the microscope to determine whether they were dead or not. If the two operators gave different results, the survival status was determined independently by a third skilled operator. The *in-vitro* culture was conducted in a climatic chamber at a temperature of 20 °C and a humidity of 96%. During the observation, the glass slides were transported in a wet box to ensure the high humidity.

### 4. Statistics

In this experiment, statistical analysis was conducted using SPSS22.0 statistical software, normality test was conducted using Shapiro-Wilk test, and homogeneity of variance test was conducted using Bartlett's test. The data with normal distribution and homogeneous variance were subjected to One-way ANOVA, and when there was statistical significance, Bonferroni's method was used for pairwise comparison among the groups; when the data did not converge to normal distribution, the Kruskal-Wallis test was adopted for statistical analysis with a test criterion α of 0.05.

### 5. Results:

### Effects of compounds on survival time of D. folliculorum

The *in-vitro* culture of the *Demodex* mites was conducted in an environment at a humidity of 96% and a temperature of 20 °C. The survival of the *Demodex* mites was observed dynamically and the survival time was recorded. The results are shown in Table 2. Compared with the negative control group receiving 10% DMSO, the survival time of the *Demodex* mites *in vitro* was significantly shorter in the taraxerol group (37.91 ± 20.96 vs. 80.79 ± 25.34, P = 0.006), the diosmetin group (35.92 ± 14.82 vs. 80.79 ± 25.34, P = 0.001), the taraxasterol acetate group (37.91 ± 20.96 vs. 80.79 ± 25.34, P < 0.001) and the gentiopicroside group (37.31 ± 36.06 vs. 80.79 ± 25.34, P < 0.001), while the survival time of the *Demodex* mites in the groups of other isolated compounds did not show significant differences (P > 0.05) as compared with the control group.

**Table 2: Effects of compounds on survival time of D. folliculorum in vitro**

| No. | Isolated compound | Survival time (hours) | Number of *Demodex* mites (N) |
|---|---|---|---|
| Control | 10%DMSO | 80.79 ±25.34 | 36 |
| 1 | Taraxasterol | 68.28±31.30 | 6 |
| 2 | Taraxerol | 37.91±20.96** | 11 |
| 3 | Gentiopicroside | 37.31±36.06*** | 15 |
| 4 | Taraxerone | 62.06±28.97 | 8 |
| 5 | Diosmetin | 35.92±14.82** | 10 |
| 6 | Loganic acid | 61.56±22.84 | 8 |
| 7 | Sweroside | 60.79±25.98 | 7 |
| 8 | Roburic acid | 63.41±12.20 | 9 |
| 9 | Taraxasterol acetate | 34.14±22.96*** | 13 |
| 10 | Taraxeryl acetate | 70.57±27.45 | 12 |
| 11 | Lupenone | 66.25±31.22 | 5 |
| 12 | Swertiamarin | 59.68±10.97 | 7 |

Multinle comnarisons were conducted using Duncan's test; as compared with the control group, P*: p < 0.05, P**: p < 0.01, P***: p < 0.001.

Only taraxerol and taraxasterol acetate are used according to the claimed invention. Any other compounds are described for reference only.

## Claims

1. A compound or a salt thereof, or a solvate thereof for use in preventing and/or treating a disease caused by a *Demodex* spp. infestation, by controlling or killing *Demodex spp.* on or in a human or an animal, wherein the compound is taraxerol or taraxasterol acetate.

2. Non-therapeutic use of a compound or a salt thereof, or a solvate thereof, for controlling or killing *Demodex* spp., wherein the compound is taraxerol or taraxasterol acetate.

3. The compound for use according to claim 1 or the use according to claim 2, wherein the compound is taraxerol.

4. The compound for use according to claim 1 or the use according to claim 2, wherein the compound is taraxasterol acetate.

5. The compound for use according to any of claims 1, 3 and 4 or the use according to any one of claims 2-4 wherein the *Demodex spp.* are *Demodex folliculorum* or *Demodex brevis.*

6. The compound for use according to any one of claims 1 and 3-5, wherein the disease is selected from: an ocular disease, a skin disease and an allergic disease.

7. The compound for use according to claim 6, wherein the disease is an ocular disease, preferably selected from: blepharitis marginalis, meibomian gland dysfunction, tarsitis, madarosis, abnormal eyelash alignment, glaucoma, cataract, ocular folliculitis, conjunctivitis, blepharoconjunctivitis, pterygium, keratitis, eyelid laxity and ectropion, basal cell carcinoma of eyelid, xerophthalmia and chalazion.

8. The compound for use according to claim 6 or claim 7, wherein the disease is a skin disease, preferably selected from: seborrhoeic dermatitis, acne, acne rosacea, pityriasis folliculorum, perioral dermatitis, demodicosis, gaile and basal cell carcinoma.

9. The compound for use according to any one of claims 6-8, wherein the disease is an allergic disease, preferably selected from: allergic asthma, allergic rhinitis and allergic dermatitis.

10. The compound for use according to claim 6 or claim 7, wherein the disease is an ocular disease, and the compound is administered to eyes of a subject in need thereof.

11. The compound for use according to claim 10, wherein the compound is administered in a form of an eye drop, an eye ointment, an ophthalmic gel, an ophthalmic emulsion, an ophthalmic suspension, an ophthalmic film, a collyrium or an intraocular injection.

12. The compound for use according to any one of claims 1 and 3-5, for use in a topical preparation, preferably an ophthalmic preparation or a skin topical preparation;
preferably, the ophthalmic preparation is selected from: an eye drop, an eye ointment, an ophthalmic gel, an ophthalmic emulsion, an ophthalmic suspension, an ophthalmic film, a collyrium and an intraocular injection;
preferably, the skin topical preparation is selected from: an aerosol, a powder, a lotion, a tincture, a liniment, a film coating agent, an ointment, a gel, a paste and an emulsion.

13. The use according to any one of claims 2-5 in cosmetics;
preferably, the cosmetics is in a form selected from: a facial cleanser, a soap, a skin softener, a toner, a skin care lotion, a jelly lotion, a facial cream, a sunscreen cream, an essence, a facial mask, a gel, a foundation, a scrub, a neck cream, a shampoo, a shower gel, a hair conditioner, a body lotion, an eye cream, a mascara, an eyeliner powder, a cream eyeliner, an eyeliner pencil, an eye shadow powder, an eye shadow cream, an eyebrow pencil and a brow powder.

14. The use according to any one of claims 2-5 in an acaricide;
preferably, the acaricide is in a form selected from: a spray, a lotion, or a paster.

## Patentansprüche

1. Verbindung oder ein Salz davon oder ein Solvat davon zur Verwendung bei der Vorbeugung und/oder Behandlung einer Krankheit, die durch einen Befall mit *Demodex* spp. verursacht wird, durch Bekämpfung oder Abtötung von *Demodex spp*. auf oder in einem Menschen oder einem Tier, wobei die Verbindung Taraxerol oder Taraxasterolacetat ist.

2. Nichttherapeutische Verwendung einer Verbindung oder eines Salzes davon oder eines Solvats davon zur Bekämpfung oder Abtötung von *Demodex* spp., wobei die Verbindung Taraxerol oder Taraxasterolacetat ist.

3. Verbindung zur Verwendung nach Anspruch 1 oder Verwendung nach Anspruch 2, wobei die Verbindung Taraxerol ist.

4. Verbindung zur Verwendung nach Anspruch 1 oder Verwendung nach Anspruch 2, wobei die Verbindung Taraxasterolacetat ist.

5. Verbindung zur Verwendung nach einem der Ansprüche 1, 3 und 4 oder Verwendung nach einem der Ansprüche 2 bis 4, wobei die *Demodex spp*. *Demodex folliculorum* oder *Demodex brevis* sind.

6. Verbindung zur Verwendung nach einem der Ansprüche 1 und 3 bis 5, wobei die Krankheit ausgewählt ist aus: einer Augenerkrankung, einer Hauterkrankung und einer allergischen Erkrankung.

7. Verbindung zur Verwendung nach Anspruch 6, wobei die Krankheit eine Augenerkrankung ist, vorzugsweise ausgewählt aus: Blepharitis marginalis, Meibom-Drüsen-Dysfunktion, Tarsitis, Madarose, abnormer Wimpernausrichtung, Glaukom, Katarakt, okulärer Follikulitis, Konjunktivitis, Blepharokonjunktivitis, Pterygium, Keratitis, Liderschlaffheit und Ektropium, Basalzellkarzinom des Augenlids, Xerophthalmie und Chalazion.

8. Verbindung zur Verwendung nach Anspruch 6 oder Anspruch 7, wobei es sich bei der Krankheit um eine Hauterkrankung handelt, vorzugsweise ausgewählt aus: seborrhoischer Dermatitis, Akne, Akne rosacea, Pityriasis folliculorum, perioraler Dermatitis, Demodikose, Gaile und Basalzellkarzinom.

9. Verbindung zur Verwendung nach einem der Ansprüche 6 bis 8, wobei die Krankheit eine allergische Erkrankung ist, vorzugsweise ausgewählt aus: allergischem Asthma, allergischer Rhinitis und allergischer Dermatitis.

10. Verbindung zur Verwendung nach Anspruch 6 oder Anspruch 7, wobei die Krankheit eine Augenerkrankung ist und die Verbindung den Augen eines Patienten zugeführt wird, der diese benötigt.

11. Verbindung zur Verwendung nach Anspruch 10, wobei die Verbindung in Form von Augentropfen, einer Augensalbe, einem ophthalmologischen Gel, einer ophthalmologischen Emulsion, einer ophthalmologischen Suspension, einem ophthalmologischen Film, einem Collyrium oder einer intraokularen Injektion verabreicht wird.

12. Verbindung zur Verwendung nach einem der Ansprüche 1 und 3 bis 5 zur Verwendung in einem topischen Präparat, vorzugsweise einem ophthalmologischen Präparat oder einem Präparat zur topischen Anwendung auf der Haut;
vorzugsweise ist das ophthalmologische Präparat ausgewählt aus: Augentropfen, einer Augensalbe, einem ophthalmologischen Gel, einer ophthalmologischen Emulsion, einer ophthalmologischen Suspension, einem ophthalmologischen Film, einem Collyrium und einer intraokularen Injektion;
vorzugsweise ist das Präparat zur topischen Anwendung auf der Haut ausgewählt aus: einem Aerosol, einem Puder, einer Lotion, einer Tinktur, einem Einreibemittel, einem Filmüberzugsmittel, einer Salbe, einem Gel, einer Paste und einer Emulsion.

13. Verwendung nach einem der Ansprüche 2 bis 5 in Kosmetika;
vorzugsweise liegen die Kosmetika in einer Form vor, die ausgewählt ist aus: einem Gesichtsreiniger, einer Seife, einem Hautweichmacher, einem Gesichtswasser, einer Hautpflegelotion, einer Gel-Lotion, einer Gesichtscreme, einer Sonnencreme, einer Essenz, einer Gesichtsmaske, einem Gel, einer Foundation, einem Peeling, einer Halscreme, einem Shampoo, einem Duschgel, einer Haarspülung, einer Körperlotion, einer Augencreme, einer Wimperntusche, einem Eyeliner-Puder, einem cremigen Eyeliner, einem Eyeliner-Stift, einem Lidschattenpuder, einem cremigen Lidschatten, einem Augenbrauenstift und einem Augenbrauenpuder.

14. Verwendung nach einem der Ansprüche 2 bis 5 in einem Akarizid;
vorzugsweise liegt das Akarizid in einer Form vor, die ausgewählt ist aus: einem Spray, einer Lotion oder einem Klebepad.

## Revendications

1. Composé ou sel de celui-ci, ou solvate de celui-ci pour utilisation dans la prévention et/ou le traitement d'une maladie causée par une infestation par *Demodex* spp., en contrôlant ou éliminant des *Demodex* spp. sur ou chez un humain ou un animal, dans lequel le composé est le taraxérol ou acétate de taraxastérol.

2. Utilisation non thérapeutique d'un composé ou d'un sel de celui-ci, ou d'un solvate de celui-ci, pour contrôler ou éliminer des *Demodex* spp., dans lequel le composé est le taraxérol ou acétate de taraxastérol.

3. Composé pour utilisation selon la revendication 1 ou utilisation selon la revendication 2, dans lequel ou laquelle le composé est le taraxérol.

4. Composé pour utilisation selon la revendication 1 ou utilisation selon la revendication 2, dans lequel ou laquelle le composé est l'acétate de taraxastérol.

5. Composé pour utilisation selon quelconques des revendications 1, 3, et 4 ou utilisation selon l'une quelconque des revendications 2 à 4, dans lequel ou laquelle les *Demodex* spp. sont *Demodex folliculorum* ou *Demodex brevis*.

6. Composé pour utilisation selon l'une quelconque des revendications 1 et 3 à 5, dans lequel la maladie est sélectionnée parmi : une maladie oculaire, une maladie cutanée, et une maladie allergique.

7. Composé pour utilisation selon la revendication 6, dans lequel la maladie est une maladie oculaire, de préférence sélectionnée parmi : blépharite ciliaire, dysfonctionnement des glandes de Meibomius, tarsite, madarose, alignement anormal des sourcils, glaucome, cataracte, folliculite oculaire, conjonctivite, blépharoconjonctivite, ptérygium, kératite, laxité des paupières et ectropion, carcinome basocellulaire de la paupière, xérophtalmie, et chalazion.

8. Composé pour utilisation selon la revendication 6 ou la revendication 7, dans lequel la maladie est une maladie cutanée, de préférence sélectionnée parmi : dermatite séborrhéique, acné, acné rosacée, pityriasis folliculorum, dermatite périorale, démodécie, gale, et carcinome basocellulaire.

9. Composé pour utilisation selon l'une quelconque des revendications 6 à 8, dans lequel la maladie est une maladie allergique, de préférence sélectionnée parmi : asthme allergique, rhinite allergique, et dermatite allergique.

10. Composé pour utilisation selon la revendication 6 ou la revendication 7, dans lequel la maladie est une maladie oculaire, et le composé est administré à des yeux d'un sujet en ayant besoin.

11. Composé pour utilisation selon la revendication 10, dans lequel le composé est administré sous une forme d'une goutte oculaire, d'un onguent oculaire, d'un gel ophtalmique, d'une émulsion ophtalmique, d'une suspension ophtalmique, d'un film ophtalmique, d'un collyre, ou d'une injection intraoculaire.

12. Composé pour utilisation selon l'une quelconque des revendications 1 et 3 à 5, pour utilisation dans une préparation topique, de préférence une préparation ophtalmique ou une préparation topique cutanée ;
de préférence, la préparation ophtalmique est sélectionnée parmi : une goutte oculaire, un onguent oculaire, un gel ophtalmique, une émulsion ophtalmique, une suspension ophtalmique, un film ophtalmique, un collyre, et une injection intraoculaire ;
de préférence, la préparation topique cutanée est sélectionnée parmi : un aérosol, une poudre, une lotion, une teinture, une pommade, un agent de pelliculage, un onguent, un gel, une pâte, et une émulsion.

13. Utilisation selon l'une quelconque des revendications 2 à 5 dans un produit cosmétique ;
de préférence, le produit cosmétique est sous une forme sélectionnée parmi : un nettoyant facial, un savon, une lotion adoucissante cutanée, une lotion tonique, une lotion de soins cutanés, une lotion gelée, une crème faciale, une crème écran solaire, une essence, un masque facial, un gel, un fond de teint, un exfoliant, une crème pour le cou, un shampooing, un gel de douche, un après-shampooing, une lotion corporelle, une crème oculaire, un mascara, une poudre de contour des yeux, une crème de contour des yeux, un crayon de contour des yeux, une poudre fard à paupières, une crème fard à paupières, un crayon à sourcils, et une poudre à sourcils.

14. Utilisation selon l'une quelconque des revendications 2 à 5 dans un acaricide ;
de préférence, l'acaricide est sous une forme sélectionnée parmi : une pulvérisation, une lotion, ou une pâte.
